# EUROPEAN PATENT SPECIFICATION

(11) **EP 1 257 234 B1**
(45) Date of publication and mention of the grant of the patent: **29.12.2004**
(21) Application number: 01904274.6
(22) Date of filing: 09.02.2001
(51) Int. Cl.: A61F 2/06, A61B 17/11

(54) **ENDOLUMINAL GRAFT SYSTEM WITH ENDOLUMINAL SIDE BRANCH GRAFT**
ENDOLUMINALES TRANSPLANTAT-SYSTEM MIT VERZWEIGTEM ENDOLUMINALEN TRANSPLANTAT
SYSTEME DE GREFFON ENDOLUMINAL AVEC GREFFON DE RAMIFICATION LATERALE ENDOLUMINAL

(30) Priority: 11.02.2000 NL 1014364; 03.03.2000 NL 1014559
(43) Date of publication of application: 20.11.2002
(73) Proprietor: Acmhainn Limited, Dublin 8 (IE)
(72) Inventor: KALMANN, Menno, Acmhainn Limited, Dublin 8 (IE); MOLL, Franciscus Laurens, Acmhainn Limited, Dublin 8 (IE)
(74) Representative: O'Brien, John Augustine
(86) International application number: PCT/IE2001/000021
(87) International publication number: WO 2001/058385

(56) References cited:
- EP-A- 0 941 714
- WO-A-97/33532
- WO-A-98/19625
- WO-A-98/19629
- WO-A-99/36002
- WO-A-99/37218
- WO-A-99/38454
- DE-U- 29 718 201
- US-A- 5 607 444

## Description

### Introduction

The invention relates to a graft assembly incorporating an endoluminal side branch graft.

An aneurysm is a swelling in an artery that is usually caused by localised damage or weakness of the vessel wall. Such aortic aneurysms are a significant medical problem and affect a significant proportion of the population. Treatment involves replacing or reinforcing the affected segment of the artery with a graft which is typically of a pliable polymeric material such as expanded polytetrafluroethylene, woven polyester or Teflon. Aneurysms may be treated using a surgical repair technique. However the less invasive technique of endovascular grafting is becoming more widely used. In this technique a prosthetic arterial graft is placed transluminally within the lumen of the affected artery. The graft is anchored in position in the vessel, typically by using a radially expandable stent.

The aorta defines a complex tortuous passageway which has a number of side branches. There are several such branch arteries including subclavian arteries, carotid arteries, intercostal arteries, renal arteries, the superior mesenteric artery, and the inferior mesenteric artery. There are particular problems in deploying endovascular grafts in regions of the aorta from which such side branches extend because in many cases the flow of blood to the side branches must be maintained.

One technique to address this problem involves providing an endovascular graft with pre-formed openings for alignment with side branches. However, this technique is not satisfactory because a very wide range of such grafts must be made available to the clinician to cater for the various locations in the aorta at which side branches may occur. In addition, the tortuosity of the aorta varies widely from one patient to another and it is consequently very difficult to provide an appropriate graft to cater for the wide variations required.

Herein are disclosed a graft assembly and a process for using such graft assemblies which will address at least some of these problems.

Document WO 99 36002 discloses a system according to the preamble of claim 1.

Document WO 98 19629 describes a graft structure with connecting and fastening means to be used to provide a by pass from the aorta to a coronary artery.

### Statements of Invention

According to the invention there is provided an endoluminal graft system comprising:-
a primary graft for placing in a body conduit at a site having a side branch from the conduit;
a side branch graft for placing in the side branch from the conduit, the side branch graft comprising a generally tubular body having a proximal part with a proximal opening, a distal part with a distal opening, and a middle section extending between the proximal and distal parts, at least one of the proximal or distal parts having a plurality of fixing fingers for in-situ fixing the side branch graft to the primary graft at an opening in the primary graft corresponding to an opening into the side branch, the fingers extending generally longitudinally in a delivery configuration and extending generally radially outwardly in a fixing configuration; and
a stabilising stent for placing in said primary graft to urge the fixing fingers into the fixing configuration and thereby maintain the fixing fingers in the fixing configuration.

In one embodiment of the invention side branch graft comprises an integral locking means to lock the fixing fingers in the fixing configuration.

Preferably the fingers are circumferentially spaced-apart in the fixing configuration. Ideally a webbing extends between the fingers.

In one case the locking means comprises an elbow hinge.

In another case the locking means comprises an elbow hinge between the fingers and the body of the graft.

In a preferred embodiment the graft comprises anchoring means on the tubular body of the graft. Preferably the anchoring means comprises one or more hook-like elements for anchoring the graft in position in the side branch.

Desirably the graft is of PTFE, polytetrafluoroethylene.

In one case the endoluminal graft system comprises a sheath for delivery or retrieval of the graft.

In one embodiment the sheath is arrangeable over the side branch graft for introduction and/or removal of the graft from its use site, the sheath when arranged on the side branch graft retaining the fixing fingers in the delivery configuration.

Preferably the system comprises a guide for guiding the system to and from the side branch graft use site. Ideally the guide comprises a guidewire over which the system is guideable.

In a preferred embodiment the system comprises a catheter having an internal diameter substantially greater than the diameter of the side branch graft, whereby the side branch graft is insertable/removable from its use site via an access opening arranged in the catheter.

There is also disclosed herein the use of an endoluminal side branch graft and/or a endoluminal side branch system and/or an endoluminal graft assembly for connecting a fluid- flow within a catheter and a body vessel particularly a blood vessel, closed off on insertion of said catheter.

Herein there is further disclosed a method for endoluminal grafting of a body conduit at a site having a side branch comprising the steps of:-
introducing a primary graft to a site in the body conduit having a side branch;
mapping the position of the side branch which has been closed off by the primary graft;
providing an opening in the primary graft at the mapped position of the side branch;
introducing a side graft into the side branch; and
fixing the side graft to the primary graft.

Preferably the position of the side branch is mapped by rotational angiography.

Ideally the opening in the primary graft is provided by *in situ* cutting of a side opening in the graft.

The primary graft may be *in situ* cut from the inside of the primary graft.

The primary graft may be *in situ* cut from outside through the side branch.

The opening is preferably provided by laser cutting.

The side graft may be introduced through the side branch.

The side graft may be introduced through the primary graft.

In a preferred embodiment the side graft comprises fixing means for fixing the side graft to the primary graft, the fixing means having a delivery configuration and a fixing configuration, and the method includes the step of fixing the side graft to the primary graft by moving the fixing means from the delivery configuration to the fixing configuration. Preferably the fixing means is moved by an actuator and the method comprises the step of introducing the actuator to the fixing means endoluminally. Ideally the actuator comprises an actuating balloon and the method comprises the step of inflating the balloon to move the fixing means from the delivery configuration to the fixing configuration.

In another preferred embodiment the method comprises the steps of:-
introducing a stabiliser to the fixing means; and actuating the stabiliser to maintain the fixing means in the fixing configuration.

There is also disclosed herein a method for endoluminal grafting of a body conduit at a site having a side branch comprising the steps of:-
introducing a primary graft to a site in the body conduit having a side branch;
mapping the position of the side branch which has been closed off by the primary graft;
*in situ* cutting an opening in the primary graft at the mapped position of the side branch by introducing a cutter through the primary graft;
introducing a side graft through the primary graft, and into the side branch; and
through the opening in the primary graft fixing the side graft to the primary graft.

Herein there is further disclosed a method for endoluminal grafting of a body conduit at a site having a side branch comprising the steps of :-
introducing a primary graft to a site in the body conduit having a side branch;
mapping the position of the side branch which has been closed off by the primary graft;
*in situ* cutting an opening in the primary graft at the mapped position of the side branch by introducing a cutter through the side branch;
introducing a side graft through the side branch; and
fixing the side graft to the primary graft.

Utilising a device according to the present invention, the supply of blood can be kept open into blood vessels branching off from the aorta, which branched blood vessels have been closed off due to the insertion of a mother graft into the aorta to treat an aneurysm, for example.

The side branch technology of this invention may be applied in particular to a Juxta renal abdominal aortic aneurysm in which one or both of the renal arteries and/or the superior mesenteric artery and/or the inferior mesenteric artery and/or coeliac trunk are provided with side branch grafts according to the invention. This is an especially difficult aneurysm to treat intraluminally because access is only available through the main artery. It is not possible to gain access to the side branches working from the outside in as the side branches lead to body organs. Therefore, in this instance the/or each side branch graft is fitted by gaining access through the primary or mother graft.

The side branch technology of the invention may also be applied particularly in treating an aneurysm in which at least some of the side branches are accessible from the outside. Such side branches include the subclavian and carotid(?) arteries. In these cases a guidewire may be readily placed in the side branches prior to deployment of a primary graft to provide rapid access for forming an opening in the primary graft and for deploying a side branch graft.

The side branch technology can also be used to simplify the grafting procedure at furcations in the artery, particularly an abdominal aortic aneurysm below the renal arteries. The term side branch as used in this specification includes such furcations.

It will be appreciated that the side branch technology of the invention may be used for grafting any suitable aneurysm. Indeed, for certain aneurysms the outside-in technique may be used to graft one or more side branches and the inside- out technique may be used to graft other side branches.

The side branch connection can be made very quickly using the device and techniques of the invention. This is particularly important in order to maintain oxygenated blood supply to vital organs.

### Brief Description of the Drawings

The invention will be more clearly understood from the following description thereof given by way of example only with reference to the accompanying drawings in which:
Fig. 1 shows a stent in an unfolded position;
Fig. 2 shows a partially cut away perspective view of a part of the assembly according to the present invention;
Fig. 3 shows a partially cut away perspective view of a side branch graft of a graft system according to the present invention, wherein the fixing positioning means are opened;
Fig. 4 shows a second embodiment of a side branch graft for present invention;
Fig. 5 shows a second preferred embodiment of a side branch graft of a graft system according to the present invention;
Figs. 6 to 13 show successively partially cut away views of insertion of the device according to the present invention into a blood vessel branching off from the aorta, which has suffered an aneurysm;
Fig. 14 shows a partially cut away perspective view of a second manner of insertion of the assembly according to the present invention.
Figs. 15 and 16 show partially cut away side views of a further preferred embodiment of a side branch graft of a graft system according to the present invention.
Figs. 17 to 22 are perspective, partially cut-away views of steps in a side branch grafting technique in which a side branch graft is placed in a side branch by access through a main graft;
Figs. 23 to 28 are perspective, partially cut-away views of steps in a side branch grafting technique in which a side branch graft is placed in a side branch by access through the side branch; and
Figs. 29 to 31 are perspective, partially cut-away views of steps in another application of the side branch grafting technique in which a side branch graft is placed in a side branch by access through the side branch.

### Detailed Description

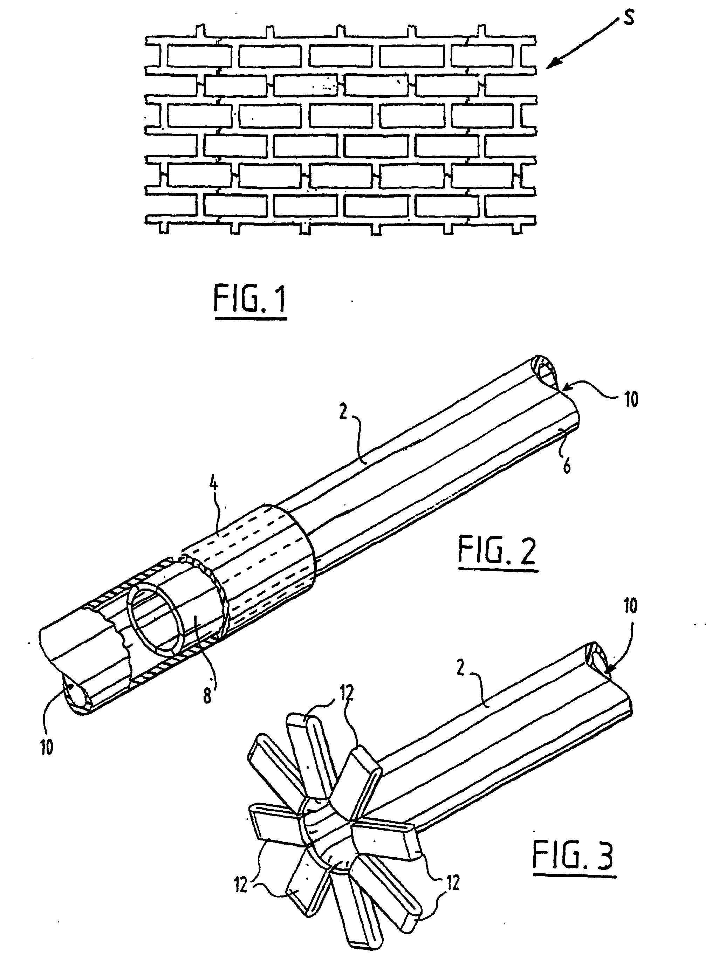

The device according to the present invention comprises a flat stent section S (see Fig. 1), which may be rolled up and covered with a graft to provide a generally cylindrical side branch graft device 2 (see Fig. 2).

An assembly according to the present invention comprises the cylindrical connecting graft device 2 and a sheath 4 arranged thereover.

The graft device 2 has a distal part 6, a proximal part 8 and an internal fluid channel 10 extending through the graft device 2.

The proximal part 8 is provided with eight separate finger sections 12, which are pretensioned to assume an open, umbrella-like position (see Fig. 3), when the sheath 4 is removed.

When the sheath 4 is arranged over the graft device 2, the fingers 12 are however closed, so that the fingers 12 are continuous with the body of the graft device 2, to provide a uniform cylindrical form in the delivery configuration.

The graft device 2 is preferably made of thin biocompatible graft material such as PTFE.

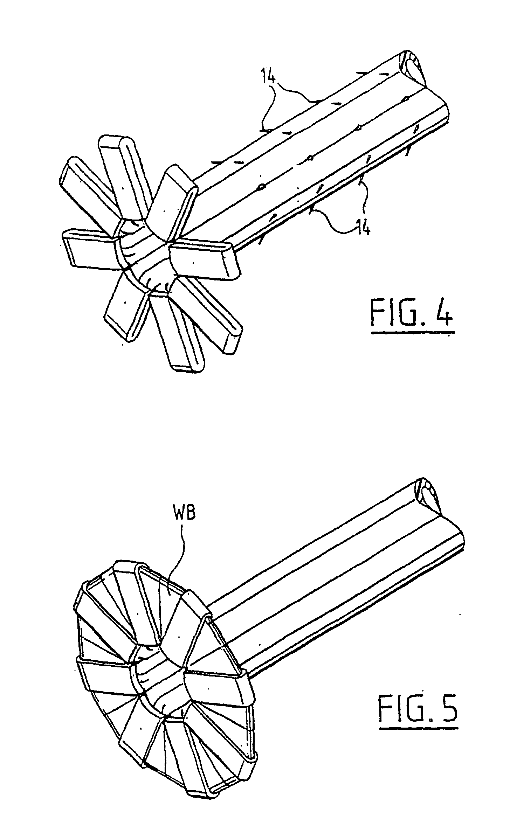

The graft device 2 can also be provided with hingable hook-like elements 14 arranged therealong to provide an extra anchoring force, when the device is arranged in position (see Fig. 4).

In a second preferred embodiment of the graft device (Fig. 5), material webbing WB is arranged between the finger sections 12. This webbing W can be made of any suitable material and further aids in anchoring the device in position and prevents unwanted matter from accumulating between the fingers.

### The device is used as follows:

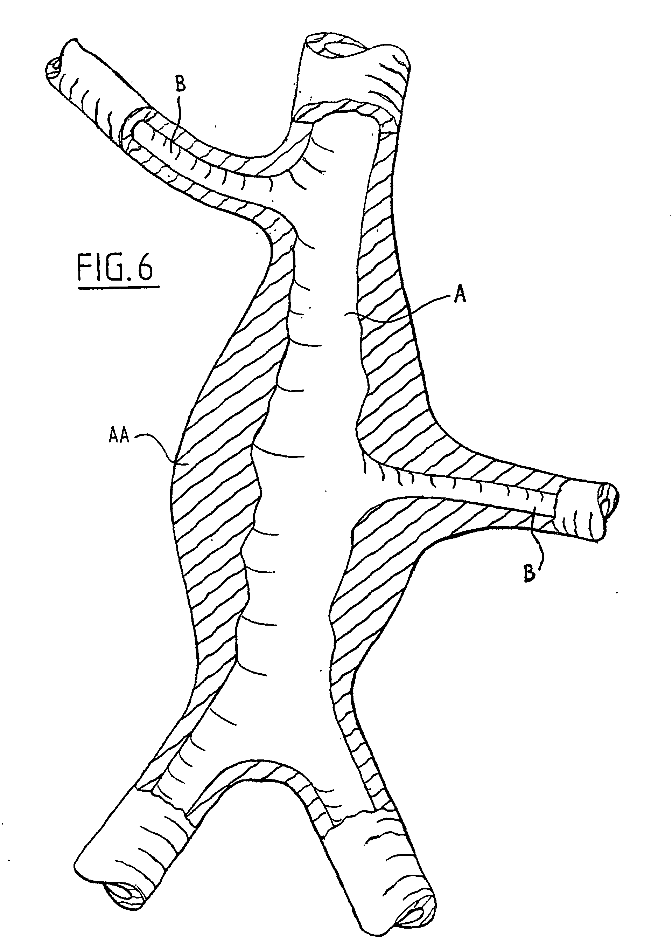

The treatment of an aortic aneurysm is often carried out by inserting a balloon catheter into the aorta to occlude the aortic aneurysm without interfering with cerebral, coronary or renal blood supply. An aneurysm, a swelling in the aorta A, is shown in Fig. 6, designated AA.

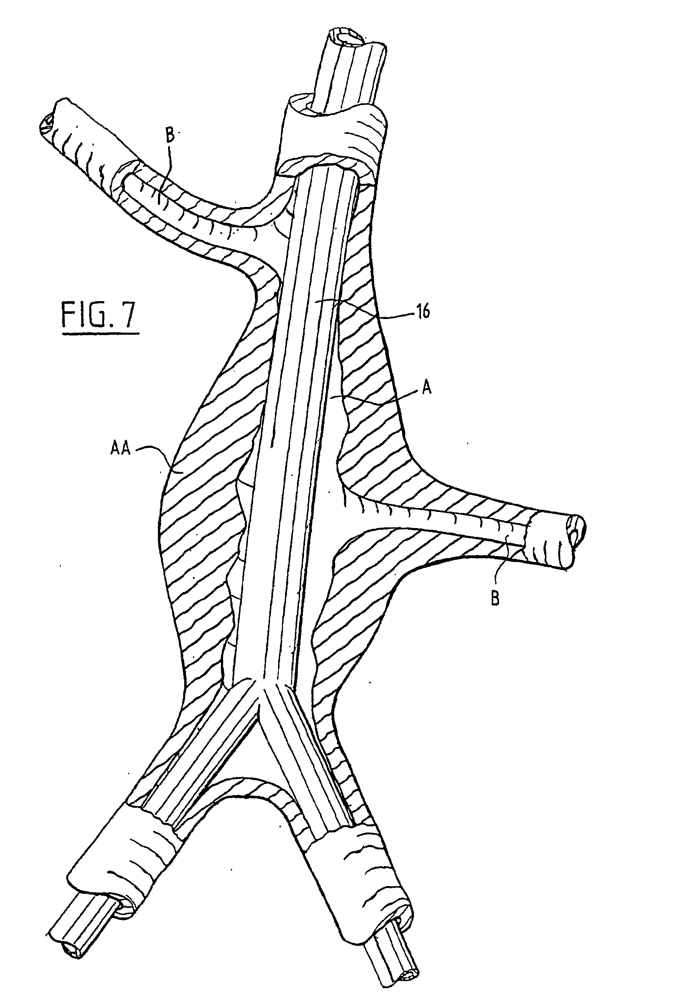

A mother or primary graft 16 is inserted via the groin into the aorta A to occlude the aneurysm (Fig. 7). On introduction of this mother graft 16, which can be done with the aid of rotational angiography, a real time three dimensional road map of the blood vessels B branching off from the aorta A is made and registered in a computer. Using these techniques the position of the side branches can be located quickly and precisely.

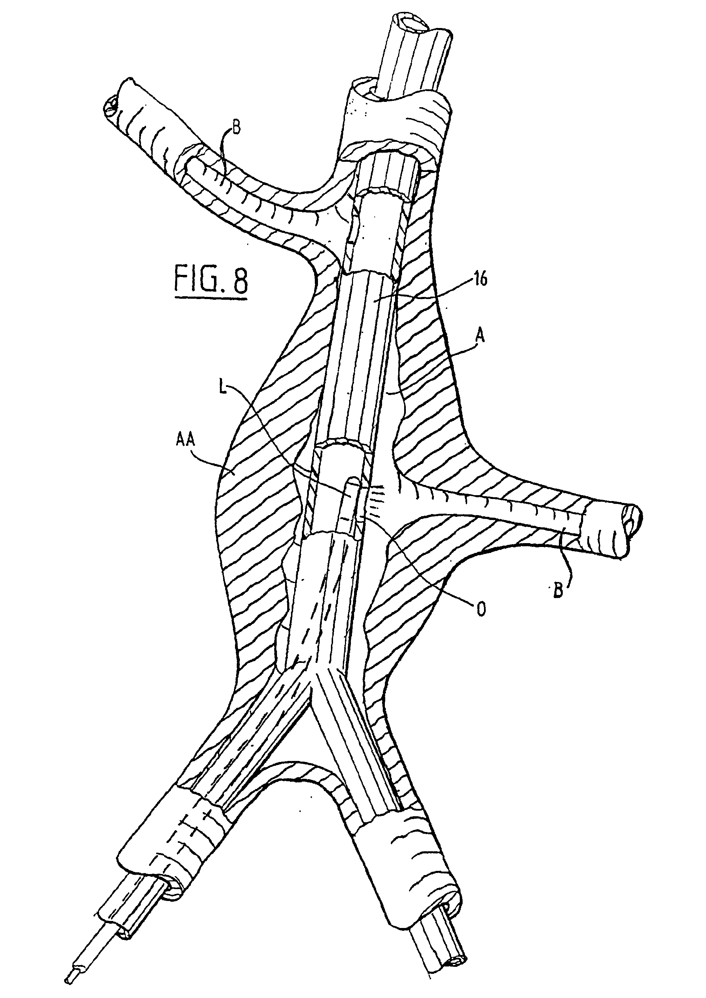

Once this road map is complete, a laser L, for example (Fig. 8) can be inserted into the mother graft 16 in order to burn openings O in the wall of the graft 16 to correspond with the computer mapped branching side vessels B (Fig. 8).

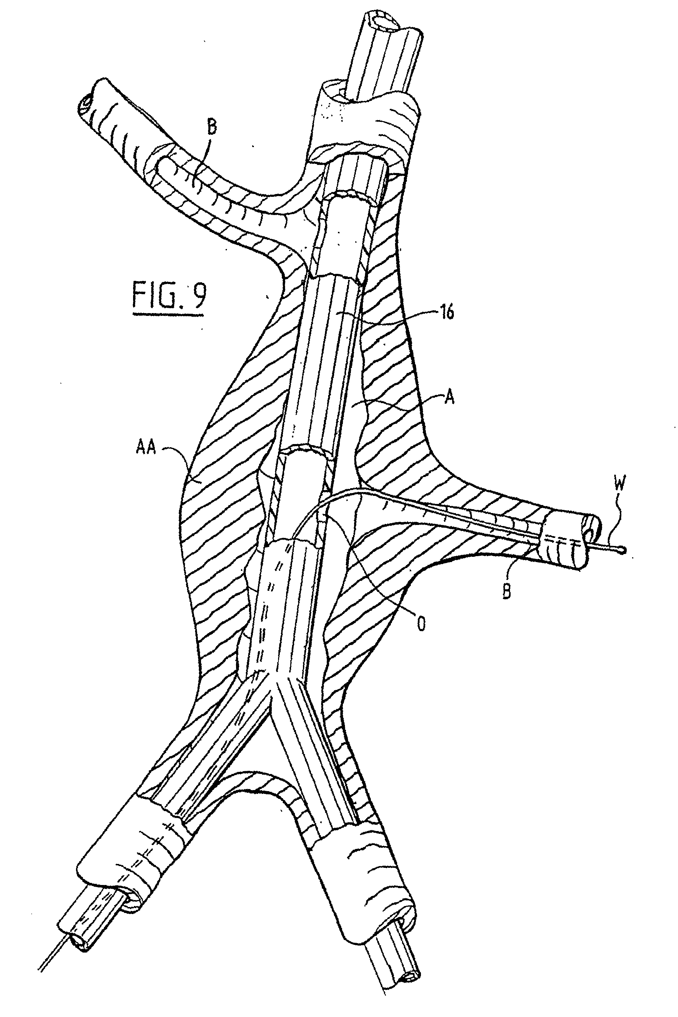

A guidewire W (Fig. 9) is then inserted through the mother graft 16 and through the opening O burned in the side wall thereof and guided through into the branched blood vessels B (Fig. 9).

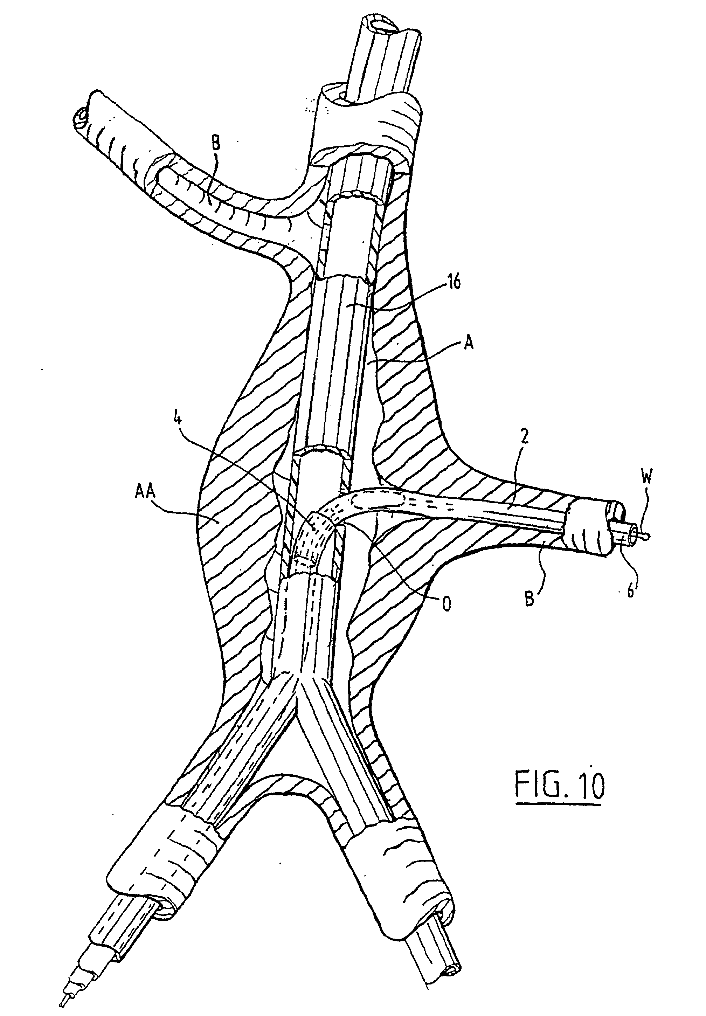

Subsequently, the assembly of the side branch graft 2 in the sheath 4 is inserted over the guide wire W through the mother graft 16, through the openings O, and into the branched blood vessel B, up until the point where an edge of the sheath 4 impinges on the periphery P of the openings O (Fig. 10). At this stage, the graft device 2 is inserted into the branched blood vessel B and the proximal part 8 thereof remains within the mother graft 16.

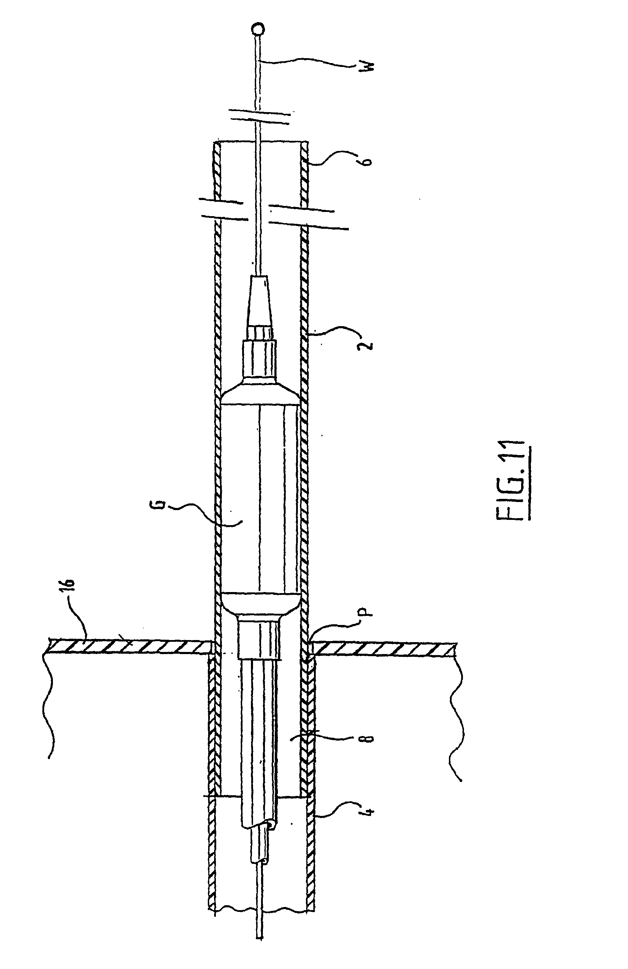

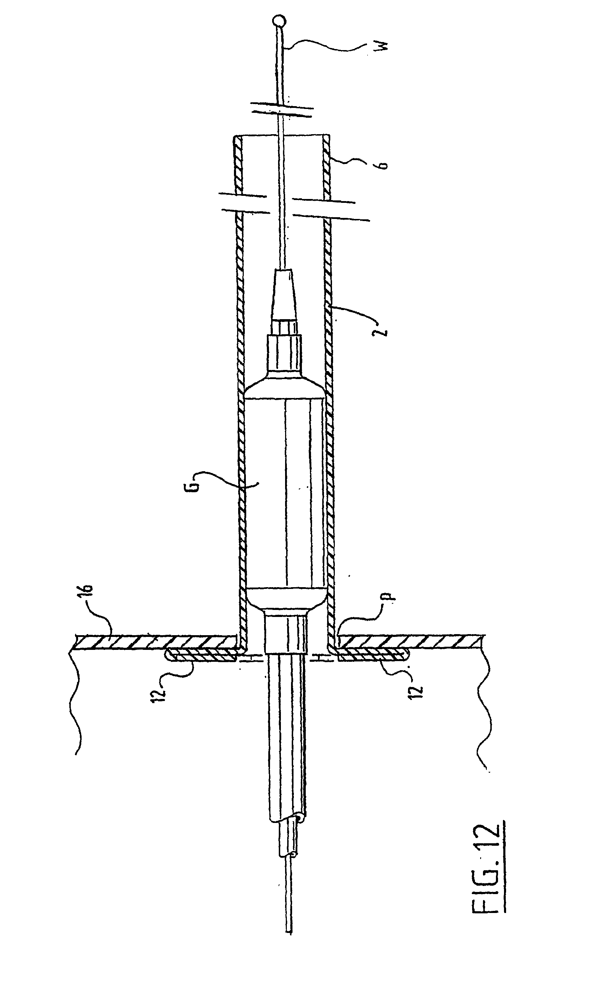

The graft device 2 can be pushed into place by means of a balloon grip G (Figs. 11 and 12) which is expanded against the interior of the graft device 2 in order to enable this to be arranged into position with a great deal of control.

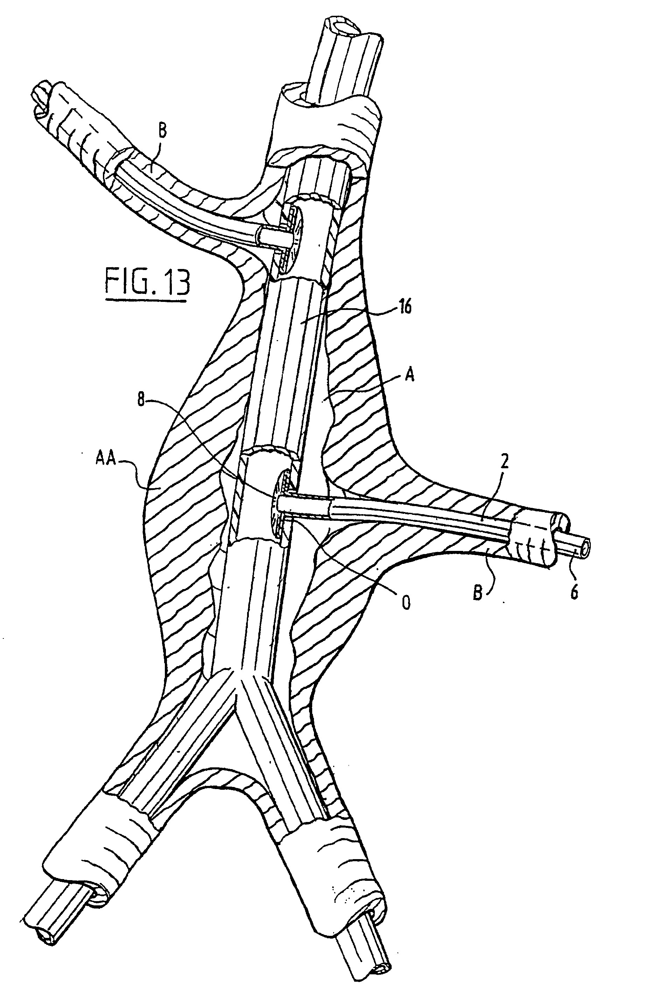

The sheath 4 is then withdrawn whereafter the fingers 12 of the proximal part 8 spring open into the pretensioned rest position (Figs. 3 to 5) to secure the proximal part 8 of the device 2, within the mother graft 16 (Figs. 12 and 13). Subsequently, the guidewire W can be removed so that the mother graft 16 has been provided with artificial side branches as it were, in the form of the side branch graft device 2 to ensure blood supply within the branched blood vessels B (Fig. 13).

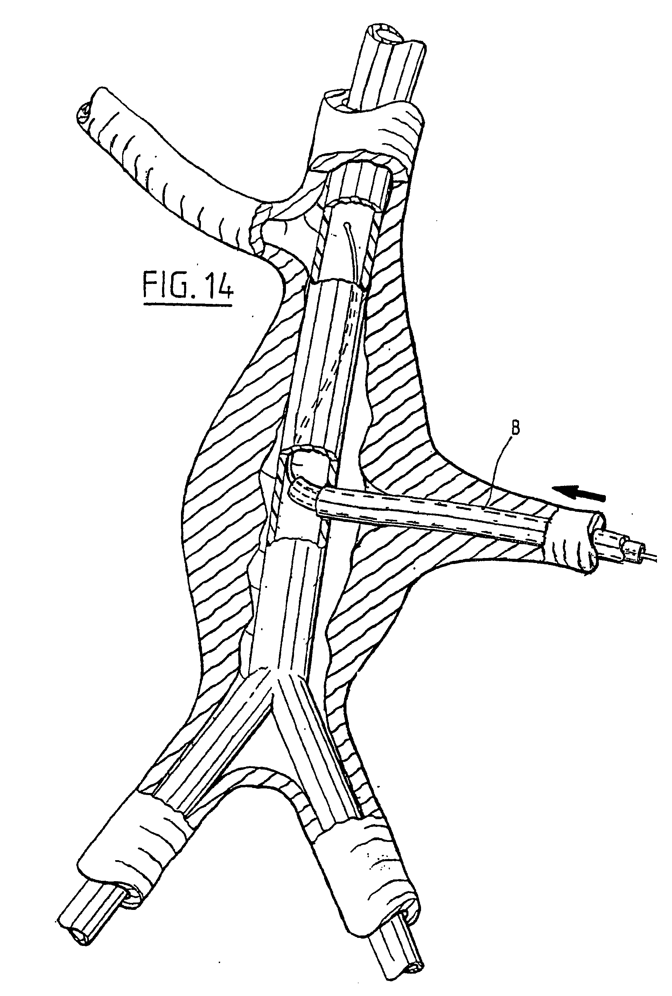

Fig. 14 perspectively shows how the assembly according to the present invention may be inserted, after an opening has been arranged in the mother graft, from the outside of the mother graft through a branched blood vessel B.

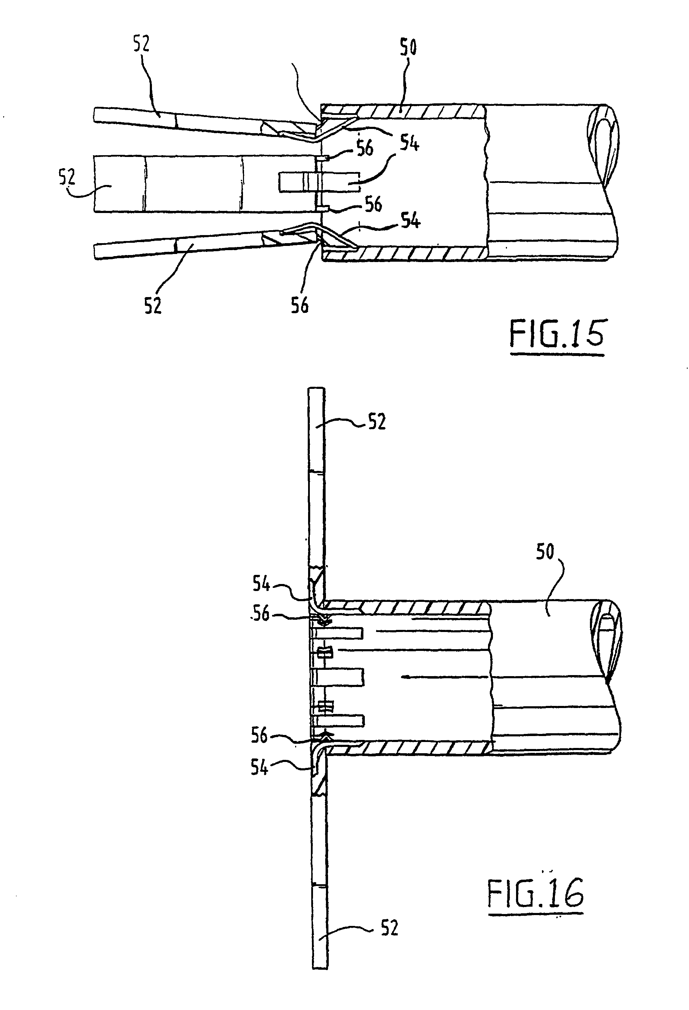

A further preferred embodiment of the present invention is shown in the partially cut away side view of Figs. 15 and 16.

In this embodiment, a proximal part of the assembly comprises a stent graft 50 and a plurality of webbed, hinged fingers 52 joined to the stent graft 50 by means of an elbow hinge 54, connected at one end to the stent graft 50 and at the other end thereof to the lower middle finger 52 and on either side thereof by two connecting strips 56, extending from the end of the finger 52 to the opening of the stent graft 50.

The fingers 52 can be clicked outwards, by means for example of an expandable balloon (not shown), so that the fingers 52 click outwards around the elbow hinges 54 and connecting strips 56 to extend at right angles to the stent graft 50. This embodiment provides a so-called bi-stable system, whereby the device can be inserted without a covering sheath in a cylindrical form, whereby thereafter the fingers 52 can be clicked open to assume their expanded anchoring position. Accordingly, the fingers of the device according to the present invention may either be self-opening, pre-tensioned, or actively openable, i.e. by means of being clicked between an open and closed position.

Thus, according to the present invention, the device, which functions as a stent for tributaries of large blood vessels, i.e. a stent for side-branches, can be arranged in position either via the side-branch into the main vessel, i.e. from "outside to inside" or via the main vessel into the side-branch, i.e. from "inside to outside".

The method chosen by the surgeon depends on the accessibility of the treatment site.

Two specific descriptions of arranging the device according to the present invention from "outside to inside" follow:

The first is an alternative for the bifurcated prosthesis, utilised when operating on an aneurysm in the stomach aorta.

According to the present invention a prosthesis can be firstly inserted via the left groin. This prosthesis is tapered whereby the wider end is attached to the aorta and the thinner end attached to the main artery in the groin. Subsequently a guidewire is inserted via the left groin through the artery and transposed therein until it abuts against the prosthesis which has just been arranged in position. An opening is then-made in the prosthesis, as described above, and the device according to the present invention is transposed over the guidewire, from outside to inside and placed in the mother prosthesis. This is a very simple and efficient manner of arranging a bifurcated prosthesis.

A second method is the treatment of an aneurysm in the arcus aorta, the artery which first extends upwards from the heart and from where side-branches extend outward to the arms and heart, and which further then bends back on itself to extend down towards the legs.

As a result of arranging this prosthesis, i.e. the mother prosthesis, also called the main catheter, in place in the arcus aorta all the side-branches to the arteries for the arms and neck/head are closed off.

At this point guidewires have already been independently inserted, by the surgeon, into the arteries leading to the arm and the neck arteries, since these are easily accessible via the neck and arms.

After the mother prosthesis has been arranged in position, these guidewires can be transposed through the arm and neck arteries until they abut against the mother prosthesis. In this manner the positioning of the openings in the mother prosthesis is directly determined.

Subsequently the openings are arranged in the mother prosthesis at these abutment points and the guidewires further transposed through these opening whereafter the side-branch stent device according to the present invention can be inserted, from the outside to the inside, over the guidewire.

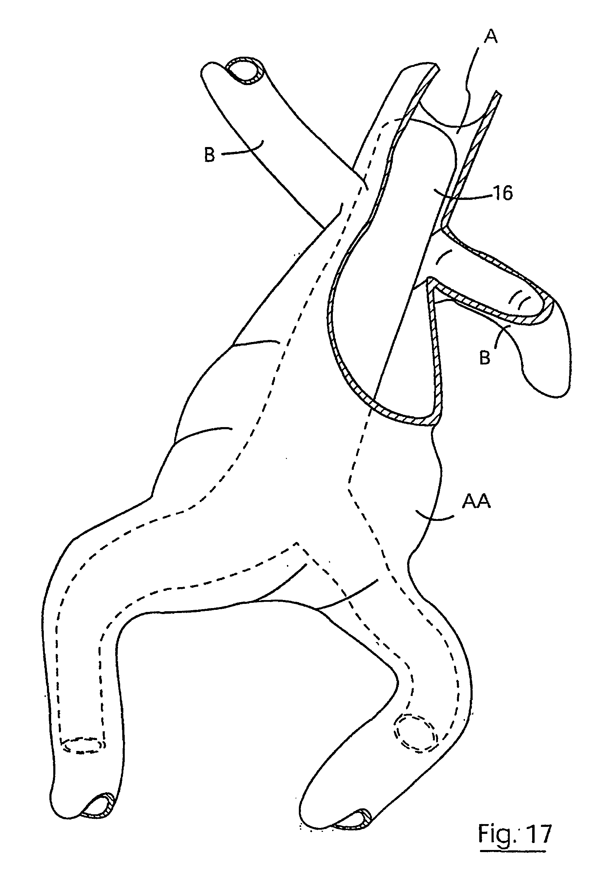

Referring to Figs. 17 to 22 there is particularly illustrated a technique for grafting side branches from the inside out. In this case the side branches B are renal arteries. However, the same technique may be used to graft other side branch arteries which are not accessible from the outside in, for example, because they terminate in an organ. Such side branches include the mesenteric artery, arteries to the spleen and liver leading from the coeliac trunk, and the lumbar artery. The drawings illustrate grafting of one side branch. The same or a similar technique is also used to provide grafts for other side branches B.

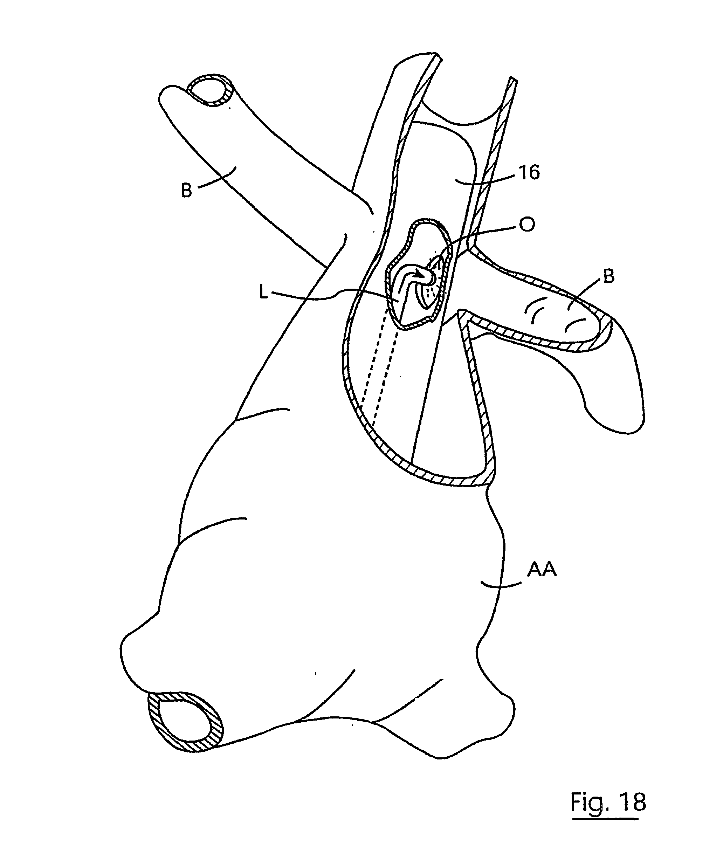

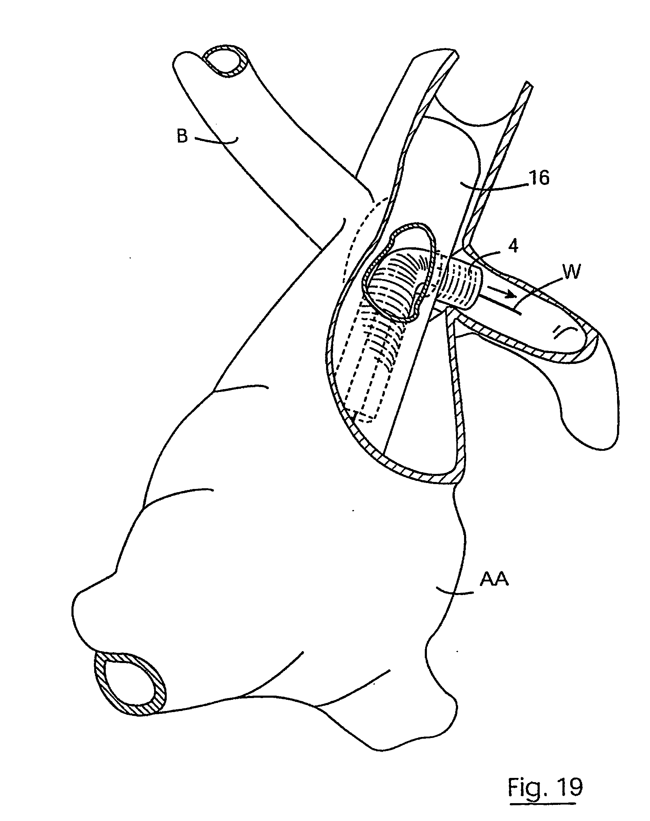

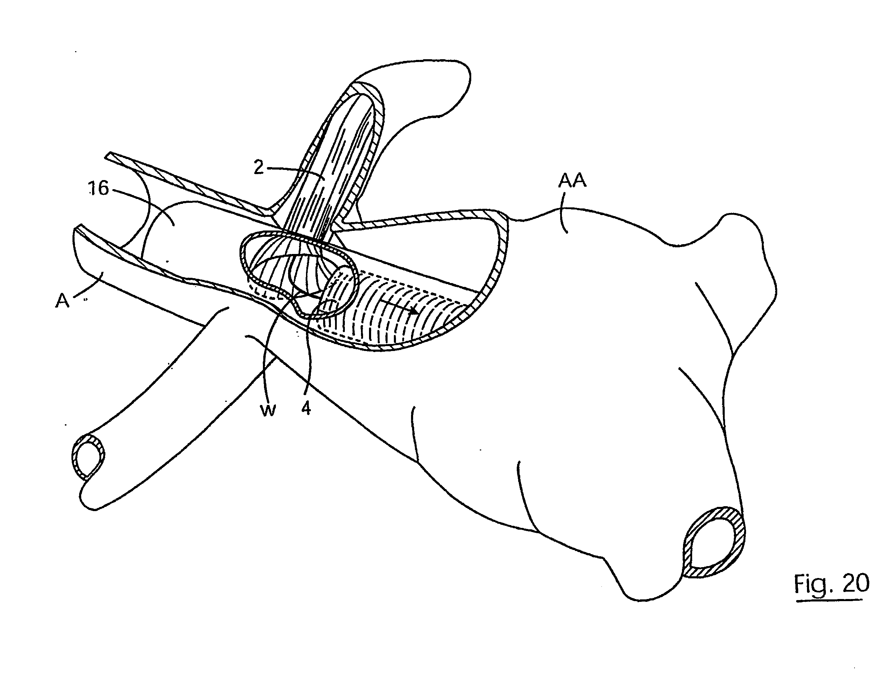

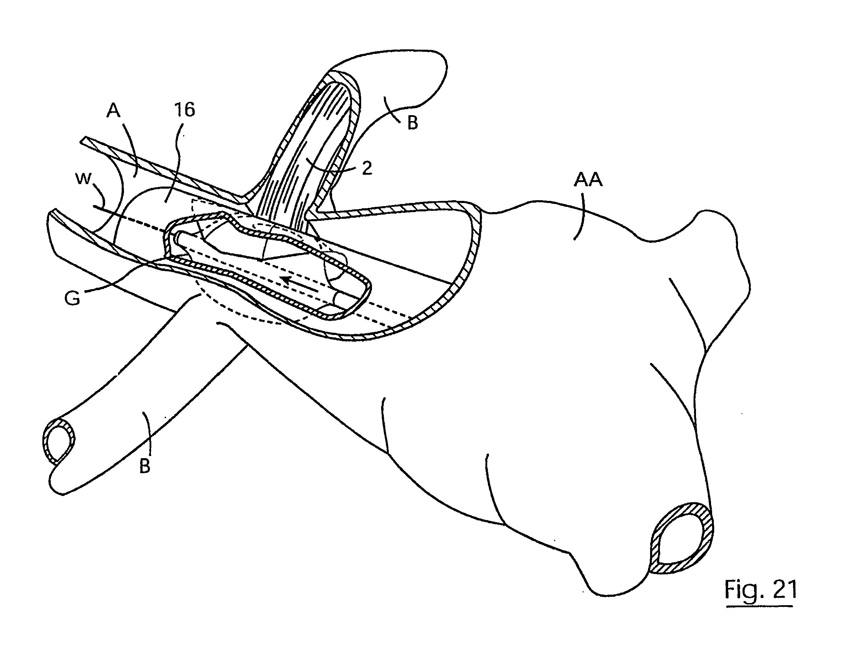

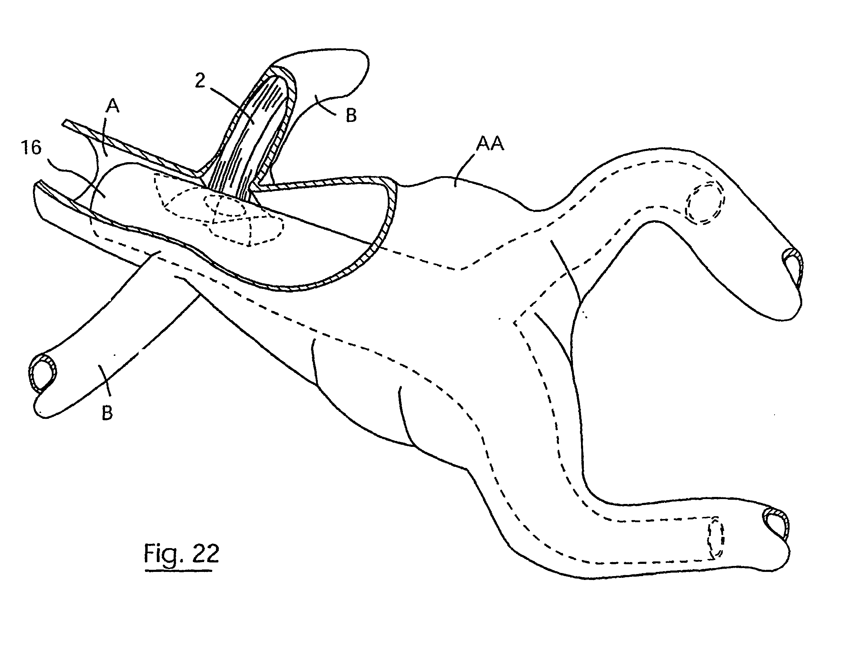

A primary or mother graft 16 is first deployed in the aorta and the position of the side branches which have been closed off by the mother graft 16 are mapped by rotational angiography (Fig. 17). A laser L is tracked over a guidewire through the mother graft 16 and aligned with the entrance to the side branch B. The mother graft 16 is then *in situ* cut by the laser to provide an opening O from the mother graft 16 into the side branch B (Fig. 18). A guidewire W is tracked into the side branch B and a sheath 4 in which a side graft/device 2 is housed is delivered over the wire into the side branch B (Fig. 19). As described above when the side graft 2 is in position the sheath 4 is withdrawn over the wire W. As the sheath 4 is withdrawn the end of the side branch graft 2 at the opening O is no longer restrained by the sheath 4 and the fingers 12 are free to move from the delivery configuration extending generally parallel to the main body of the side graft 2 to a fixing configuration in which the fingers extend radially outwardly to engage the inner surface of the mother graft 16 in the region of the opening O (Fig. 20). A balloon G may then be introduced through the mother graft 16 and expanded as illustrated in Fig. 21 to assist in fixing the side branch graft 2 in position extending from the mother graft 16. The balloon G and the guidewire W are removed to leave the side branch graft device 2 in position extending from the mother graft 16 (Fig.22)

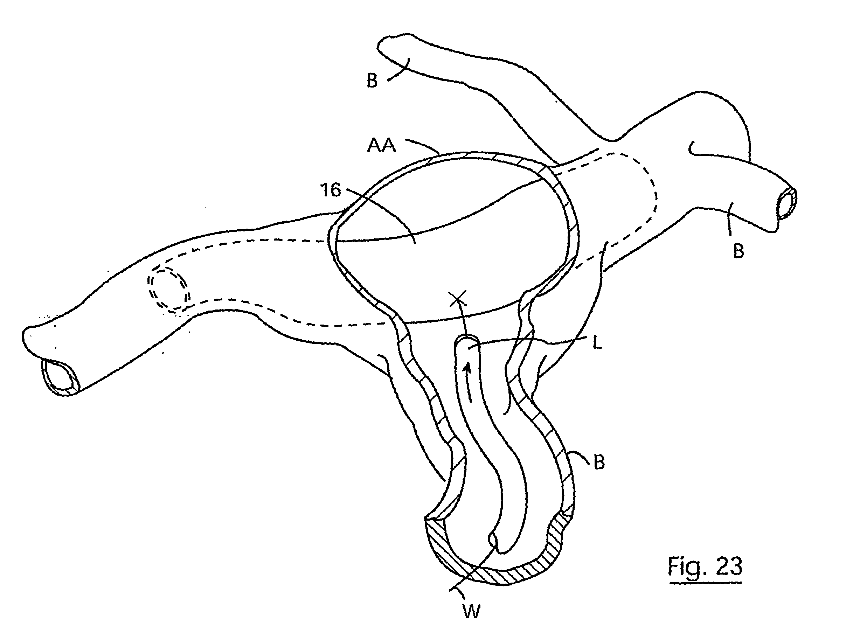

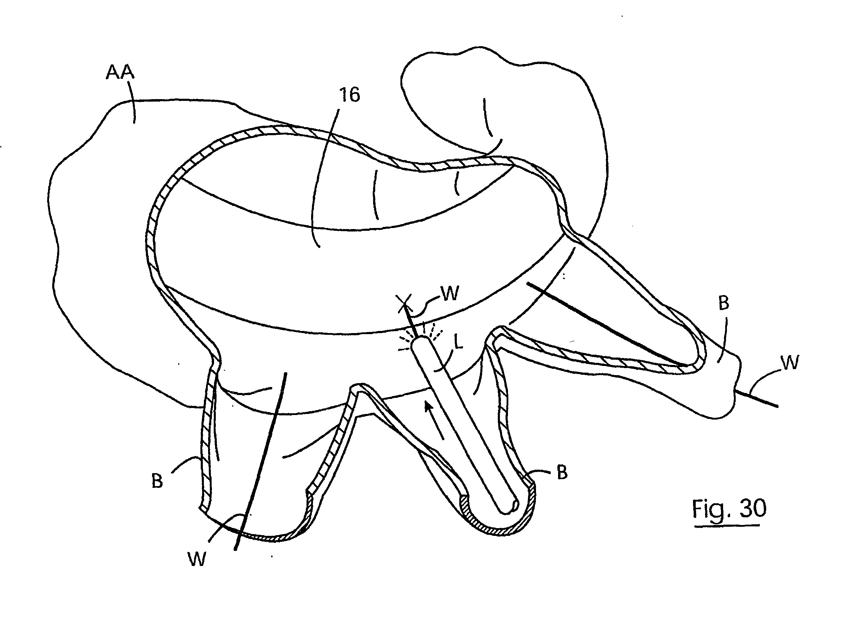

Referring now to Figs. 23 to 31 there is particularly illustrated a technique for grafting side branches from the outside in. This technique can be used in particular to provide side branch grafts in arteries which are accessible from the outside.

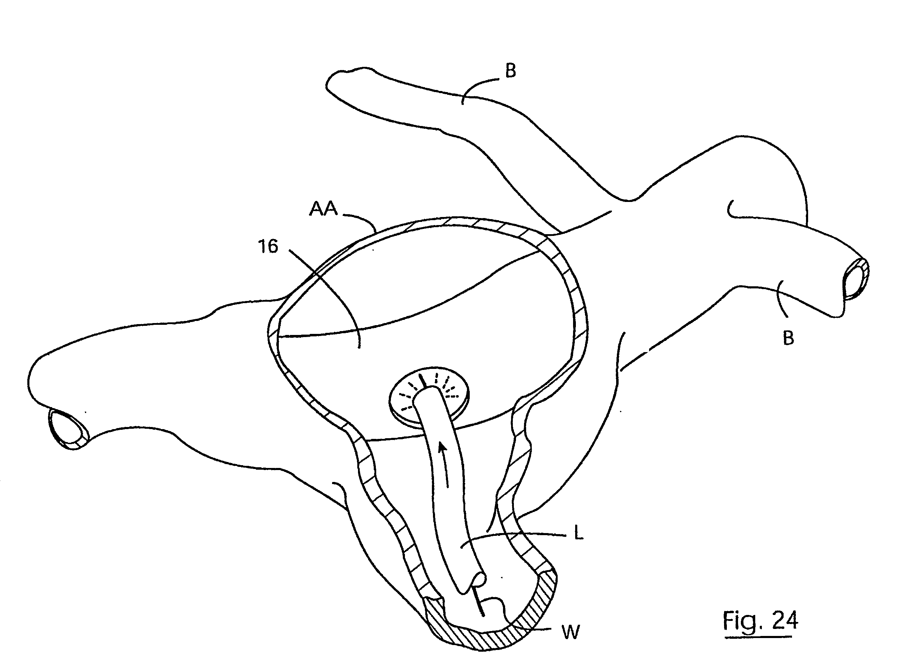

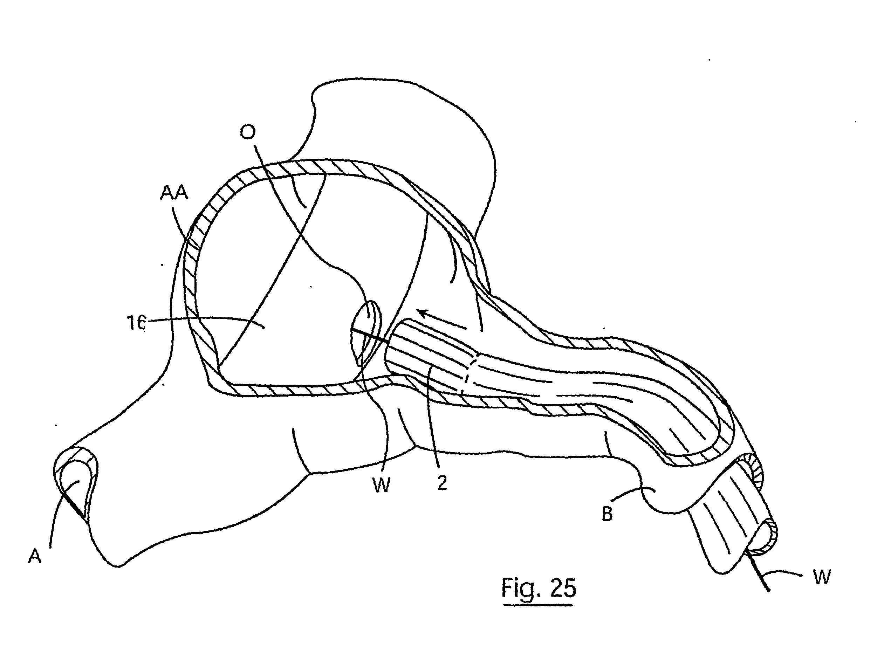

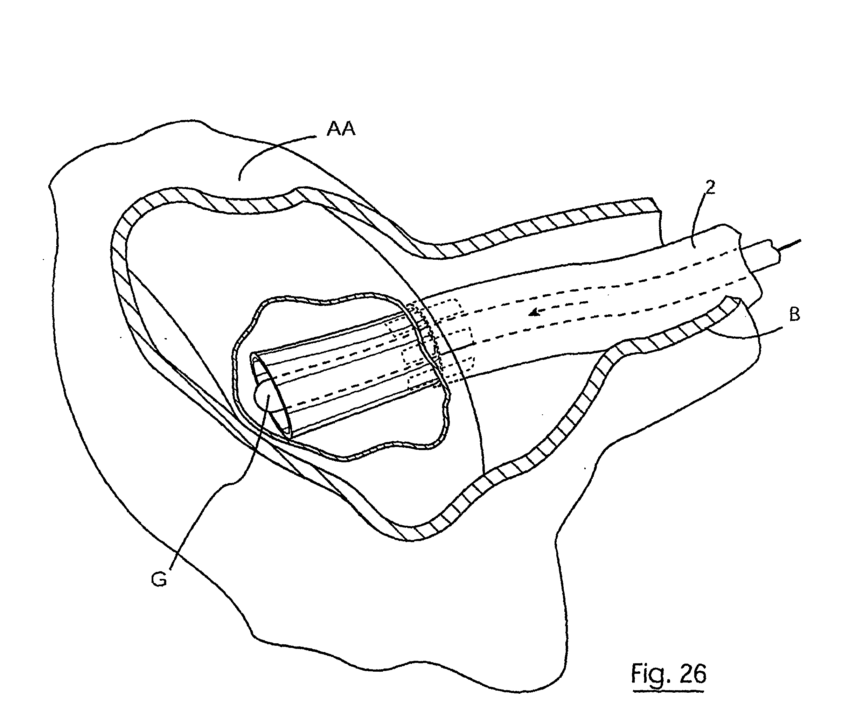

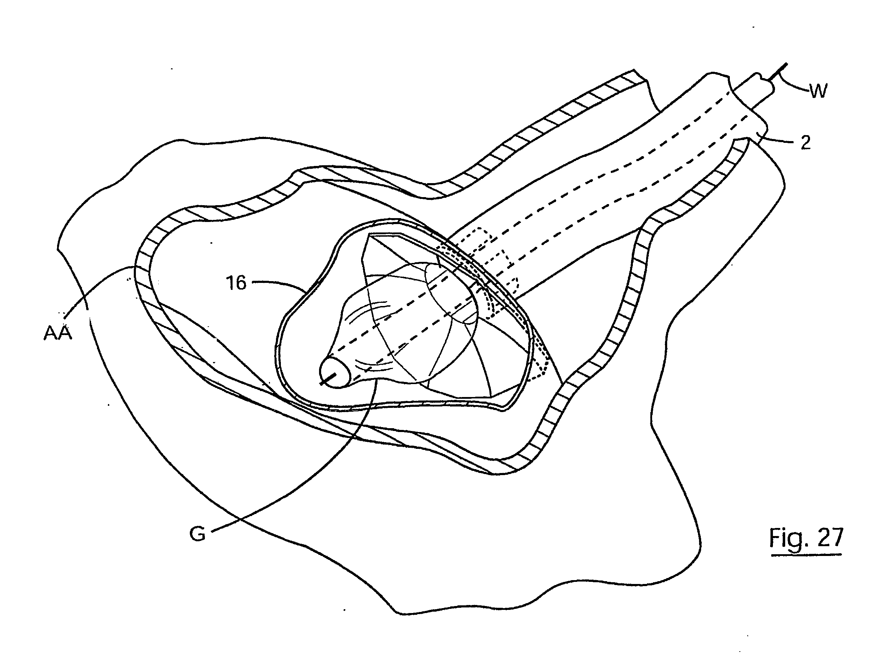

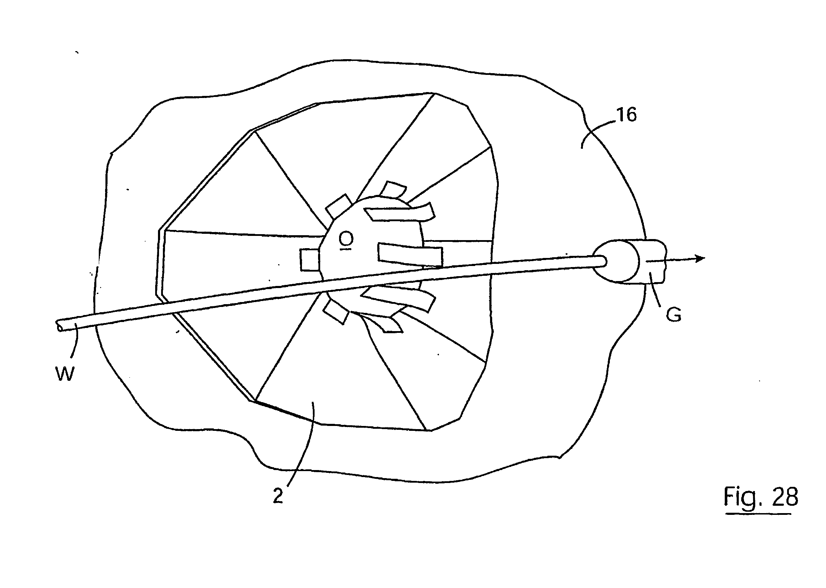

For example, and as particularly illustrated in Figs. 23 to 28 the technique can be used to provide a graft in an artery with a bifurcation in which a primary or mother graft 16 is first inserted, typically through the right groin and one end is attached to the aorta and the other end attached to the main iliac artery in the groin. A side branch graft 2 of the invention is then deployed in the other leg of the bifurcation. A guidewire W is first inserted via the left groin through the artery until it abuts against the mother graft 16 which has been placed in position. A laser L is then tracked over the guidewire W (Fig. 23) and a hole O is *in situ* cut in the mother graft 16 (Fig. 24). The laser L is removed and a side branch graft 2 is delivered over the guidewire to the opening O (Fig. 25). The distal end of the graft 2 is then inserted through the opening O (Fig. 26). The distal end of the graft 2 in this case may be of the bistable type described above and may be moved into the deployed configuration by a first balloon G delivered through the side branch (Fig. 27). To further fix the stent graft in position a second balloon may be tracked over a guidewire W through the mother graft 16 to the opening O into the side branch and inflated to press the distal end of the side branch 2 into close engagement with the inner wall of the mother graft 16. The balloon G is then deflated and withdrawn over the guidewire W as illustrated in Fig. 28.

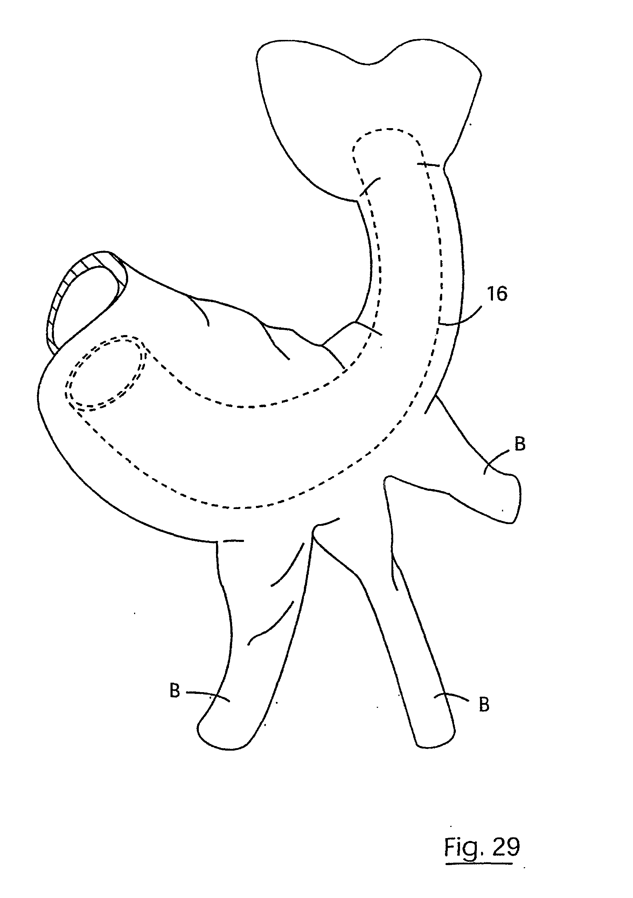

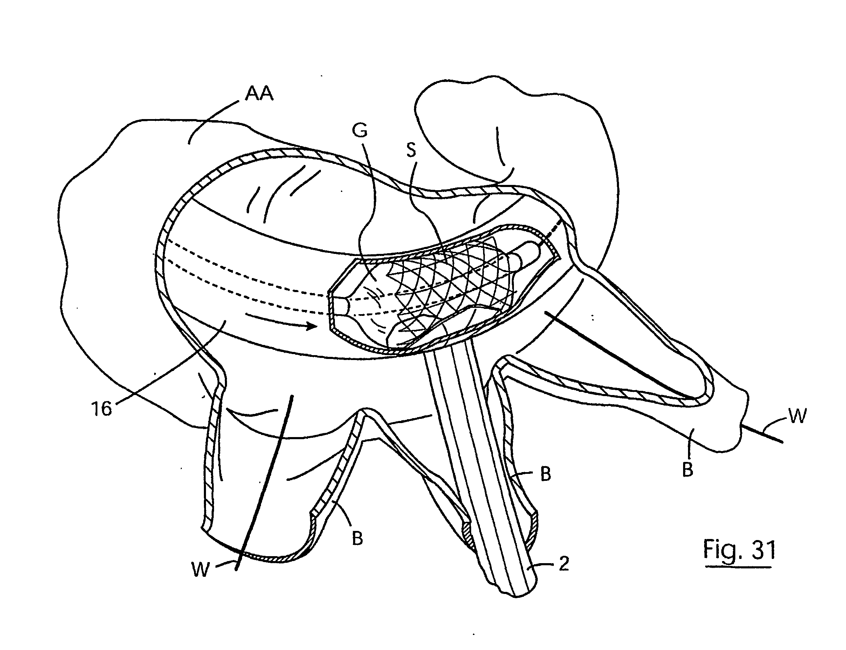

Side branch grafting of the arcus aorta as described above is illustrated in Figs. 29 to 31 utilising the outside-in technique. In this case a stent S may be deployed by a balloon G at the opening O to the side branch B when the side branch graft 2 is in position. The deployed stents provides a radially outward force on the side branch graft 2 when in position to secure the side branch 2 in close engagement with the inner wall of the mother graft 16, as illustrated in Fig. 31.

The invention is not limited to the embodiments hereinbefore described which may be varied in construction and detail within the scope of the appended claims.

## Claims

1. An endoluminal graft system comprising:-
a primary graft (16) for placing in a body conduit (A) at a site having a side branch (B) from the conduit (A);
a side branch graft (2) for placing in the side branch (B) from the conduit (A), the side branch graft (2) comprising a generally tubular body having a proximal part (8) with a proximal opening, a distal part (6) with a distal opening, and a middle section extending between the proximal (8) and distal parts (6), at least one of the proximal (8) or distal parts (6) having a plurality of fixing fingers (12) for in-situ fixing the side branch graft (2) to the primary graft (16) at an opening in the primary graft (16) corresponding to an opening into the side branch (8), the fingers (12) extending generally longitudinally in a delivery configuration and extending generally radially outwardly in a fixing configuration; **characterised in that** it further comprises
a stabilising stent for placing in said primary graft (16) to urge the fixing fingers (12) into the fixing configuration and thereby maintain the fixing fingers (12) in the fixing configuration.

2. An endoluminal graft system as claimed in claim 1 wherein the fixing fingers (12) are provided at the proximal part (8) of the side branch graft (2).

3. An endoluminal graft system as claimed in 1 wherein the fixing fingers (12) are provided at the distal part (6) of the side branch graft (2).

4. An endoluminal graft system as claimed in any of claims 1 to 3 wherein the fingers (12) are circumferentially spaced-apart in the fixing configuration.

5. An endoluminal graft system as claimed in claim 4 wherein a webbing (WB) extends between the fingers (12).

6. An endoluminal graft system as claimed in any preceding claim wherein the side branch graft (2) comprises an integral locking means to lock the fixing fingers (12) in the fixing configuration.

7. An endoluminal graft system as claimed in claim 6 wherein the locking means comprises an elbow hinge (54).

8. An endoluminal graft system as claimed in claim 6 or 7 wherein the locking means comprises an elbow hinge (54) between the fingers (12) and the body of the graft (2).

9. An endoluminal graft system as claimed in any preceding claim wherein the side branch graft (2) comprises anchoring means on the tubular body of the graft (2).

10. An endoluminal graft system as claimed in claim 9 wherein the anchoring means comprises one or more hook-like elements (14) for anchoring the graft (2) in position in the side branch (B).

11. An endoluminal graft system as claimed in any preceding claim wherein the side branch graft (2) is of PTFE, polytetrafluoroethylene.

12. An endoluminal graft system as claimed in any preceding claim comprising a sheath (4) for delivery or retrieval of the side branch graft (2).

13. An endoluminal graft system as claimed in claim 12 wherein the sheath (4) is arrangeable over the side branch graft (2) for introduction and/or removal of the graft (2) from its use site, the sheath (4) when arranged on the side branch graft (2) retaining the fixing fingers (12) in the delivery configuration.

14. An endoluminal graft system as claimed in claim 12 or 13 comprising a guide for guiding the system to and from the side branch graft (B) use site.

15. An endoluminal graft system as claimed in claim 14 wherein the guide comprises a guidewire (W) over which the system is guideable.

16. An endoluminal graft system as claimed in any preceding claim comprising a catheter having an internal diameter substantially greater than the diameter of the side branch graft (2), the side branch graft (2) being insertable/removable from its use site via an access opening arranged in the catheter.

## Patentansprüche

1. Endoluminales Graft-System, umfassend:
ein Primär-Graft (16) zum Platzieren in einem Körperkanal (A) an einer Stelle mit einem Seitenabzweig (B) von dem Kanal (A);
ein Seitenabzweig-Graft (2) zum Platzieren in dem Seitenabzweig (B) von dem Kanal (A), wobei das Seitenabzweig-Graft (2) einen allgemein röhrenförmigen Körper mit einem nahen Teil (8) mit einer nahen Öffnung, einem entfernten Teil (6) mit einer entfernten Öffnung, und einem Mittelabschnitt aufweist, der sich zwischen dem nahen (8) und entfernten Teil (6) erstreckt, wobei mindestens einer des nahen (8) oder entfernten Teils (6) eine Mehrzahl von Fixierfingem (12) für Fixierung des Seitenabzeig-Graft (2) in-situ an dem Primär-Graft (16) an einer Öffnung in dem Primär-Graft (16) umfasst, die einer Öffnung in den Seitenabzweig (B) entspricht, wobei sich die Finger (12) allgemein in Längsrichtung in einer Zuführkonfiguration erstrecken und sich allgemein radial nach außen in einer Fixierkonfiguration erstrecken, **dadurch gekennzeichnet** es ferner aufweist:
ein Stabilisierungsstent zum Platzieren in dem genannten Primär-Graft (16), um die Fixierfinger (12) in die Fixierkonfiguration zu drücken und dadurch die Fixierfinger (12) in der Fixierkonfiguration zu halten.

2. Endoluminales Graft-System nach Anspruch 1, bei dem die Fixierfinger (12) an dem nahen Teil (8) des Seitenabzweig-Graft (2) vorgesehen sind.

3. Endoluminales Graft-System nach Anspruch 1, bei dem die Fixierfinger (12) an dem entfernten Teil (6) des Seitenabzweig-Graft (2) vorgesehen sind.

4. Endoluminales Graft-System nach einem der Ansprüche 1 bis 3, bei dem die Finger (12) am Umfang in der Fixierkonfiguration beabstandet sind.

5. Endoluminales Graft-System nach Anspruch 4, bei dem sich ein Netz (WB) zwischen den Fingern (12) erstreckt.

6. Endoluminales Graft-System nach einem vorhergehenden Anspruch, bei dem das Seitenabzweig-Graft (2) ein integriertes Verriegelungsmittel zum Verriegeln der Fixierfinger (12) in der Fixierkonfiguration aufweist.

7. Endoluminales Graft-System nach Anspruch 6, bei dem das Verriegelungsmittel ein Winkelgelenk (54) aufweist.

8. Endoluminales Graft-System nach Anspruch 6 oder 7, bei dem das Verriegelungsmittel ein Winkelgelenk (54) zwischen den Fingern (12) und dem Körper des Graft (2) aufweist.

9. Endoluminales Graft-System nach einem vorhergehenden Anspruch, bei dem das Seitenabzweig-Graft (2) Verankerungsmittel an dem röhrenförmigen Körper des Graft (2) aufweist.

10. Endoluminales Graft-System nach Anspruch 9, bei dem das Verankerungsmittel ein oder mehrere hakenartige Elemente (14) zum Verankern des Graft (2) in Position in dem Seitenabzweig (B) aufweist.

11. Endoluminales Graft-System nach einem vorhergehenden Anspruch, bei dem das Seitenabzweig-Graft (2) aus PTFE, Polytetrafluorethylen besteht.

12. Endoluminales Graft-System nach einem vorhergehenden Anspruch, das eine Hülle (4) für Zuführung oder Entfernung des Seitenabzweig-Graft (2) aufweist.

13. Endoluminales Graft-System nach Anspruch 12, bei dem die Hülle (4) über dem Seitenabzweig-Graft (2) für Einführung und/oder Entfernung des Graft (2) von seiner Verwendungsstelle angeordnet sein kann, wobei die Hülle (4) bei Anordnung auf dem Seitenabzweig-Graft (2) die Fixierfinger (12) in der Zuführkonfiguration festhält.

14. Endoluminales Graft-System nach Anspruch 12 oder 13, das eine Führung zum Führen des Systems zu und aus der Verwendungsstelle des Seitenabzweig-Graft (B) aufweist.

15. Endoluminales Graft-System nach Anspruch 14, bei dem die Führung einen Führungsdraht (W) aufweist, über den das System geführt werden kann.

16. Endoluminales Graft-System nach einem vorhergehenden Anspruch, das ein Katheter mit einem Innendurchmesser aufweist, der im wesentlich größer als der Durchmesser des Seitenabzweig-Graft (2) ist, wobei das Seitenabzweig-Graft (2) über eine in dem Katheter angeordnete Zugangsöffnung in seine Verwendungsstelle eingeführt/ aus dieser entfernt werden kann.

## Revendications

1. Système de greffe endoluminale comprenant :
une greffe principale (16) à placer dans un conduit corporel (A) au niveau d'un site ayant un branchement latéral (B) à partir du conduit (A) :
une greffe de branchement latéral (2) à placer dans le branchement latéral (B) à partir du conduit (A), la greffe de branchement latéral (2) comprenant un corps généralement tubulaire ayant une partie proche (8) avec une ouverture proche, une partie distale (6) avec une ouverture distale, et une section centrale s'étendant entre les parties proche (8) et distale (6), au moins l'une des parties proche (8) ou distale (6) ayant une pluralité de doigts de fixation (12) pour la fixation in situ de la greffe de branchement latéral (2) sur la greffe principale (16) au niveau d'une ouverture dans la greffe principale (16) correspondant à une ouverture dans le branchement latéral (B), les doigts (12) s'étendant généralement longitudinalement dans une configuration de pose et s'étendant généralement radialement vers l'extérieur dans une configuration de fixation ; **caractérisé en ce qu'**il comprend en outre
un stent de stabilisation à placer dans ladite greffe principale (16) afin de forcer les doigts de fixation (12) à prendre la configuration de fixation et ainsi maintenir les doigts de fixation (12) dans la configuration de fixation.

2. Système de greffe endoluminale selon la revendication 1, dans lequel les doigts de fixation (12) sont fournis au niveau de la partie proche (8) de la greffe de branchement latéral (2).

3. Système de greffe endoluminale selon la revendication 1, dans lequel les doigts de fixation (12) sont fournis au niveau de la partie distale (6) de la greffe de branchement latéral (2).

4. Système de greffe endoluminale selon l'une quelconque des revendications 1 à 3, dans lequel les doigts (12) sont espacés circonférentiellement dans la configuration de fixation.

5. Système de greffe endoluminale selon la revendication 4, dans lequel une palmure (WB) s'étend entre les doigts (12).

6. Système de greffe endoluminale selon l'une quelconque des revendications précédentes, dans lequel la greffe de branchement latéral (2) comprend un moyen de verrouillage intégré afin de verrouiller les doigts de fixation (12) dans la configuration de fixation.

7. Système de greffe endoluminale selon la revendication 6, dans lequel le moyen de verrouillage comprend une articulation coudée (54).

8. Système de greffe endoluminale selon la revendication 6 ou 7, dans lequel le moyen de verrouillage comprend une articulation coudée (54) entre les doigts (12) et le corps de la greffe (2).

9. Système de greffe endoluminale selon l'une quelconque des revendications précédentes, dans lequel la greffe de branchement latéral (2) comprend un moyen d'ancrage sur le corps tubulaire de la greffe (2).

10. Système de greffe endoluminale selon la revendication 9, dans lequel le moyen d'ancrage comprend un ou plusieurs éléments en forme de crochet (14) pour ancrer la greffe (2) en position dans le branchement latéral (B).

11. Système de greffe endoluminale selon l'une quelconque des revendications précédentes, dans lequel la greffe de branchement latéral (2) est du PTFE, polytétrafluoroéthylène.

12. Système de greffe endoluminale selon l'une quelconque des revendications précédentes, comprenant une gaine (4) pour la pose ou le retrait de la greffe de branchement latéral (2).

13. Système de greffe endoluminale selon la revendication 12, dans lequel la gaine (4) peut être disposée par-dessus la greffe de branchement latéral (2) pour l'introduction et/ou le retrait de la greffe (2) de son site d'utilisation, la gaine (4) quand elle est agencée sur la greffe de branchement latéral (2) retenant les doigts de fixation (12) dans la configuration de pose.

14. Système de greffe endoluminale selon la revendication 12 ou 13, comprenant un guide pour guider le système vers et depuis le site d'utilisation de la greffe de branchement latéral (B).

15. Système de greffe endoluminale selon la revendication 14, dans lequel le guide comprend un fil de guidage (W) sur lequel le système peut être guidé.

16. Système de greffe endoluminale selon l'une quelconque des revendications précédentes, comprenant un cathéter ayant un diamètre interne substantiellement supérieur au diamètre de la greffe de branchement latéral (2), la greffe de branchement latéral (2) pouvant être insérée/retirée de son site d'utilisation par l'intermédiaire d'une ouverture d'accès aménagée dans le cathéter.
